# EUROPÄISCHE PATENTSCHRIFT

(11) **EP 3 152 172 B1**
(45) Veröffentlichungstag und Bekanntmachung des Hinweises auf die Patenterteilung: **26.10.2022**
(21) Anmeldenummer: 15722723.2
(22) Anmeldetag: 15.05.2015
(51) Int. Cl.: C03C 3/087, C03C 3/097, C03C 10/00, A61K 6/833, A61K 6/836, C03B 32/02, C03C 4/00

(54) **VERWENDUNG VON GLASKERAMIK MIT TIEFQUARZ ALS HAUPTKRISTALLPHASE ALS DENTALMATERIAL**
USE OF GLASS CERAMIC WITH ALPHA-QUARTZ AS THE MAIN CRYSTALLINE PHASE AS DENTAL MATERIAL
UTILISATION D'UNE VITROCÉRAMIQUE AVEC DU QUARTZ ALPHA COMME PHASE CRISTALLINE PRINCIPALE COMME MATÉRIAU DENTAIRE

(30) Priorität: 16.05.2014 EP 14168719
(43) Veröffentlichungstag der Anmeldung: 12.04.2017
(73) Patentinhaber: Ivoclar Vivadent AG, 9494 Schaan (LI)
(72) Erfinder: RAMPF, Markus, 8853 Lachen (CH); DITTMER, Marc, 6800 Feldkirch (AT); RITZBERGER, Christian, CH-9472 Grabs (CH); SCHWEIGER, Marcel, CH-7000 Chur (CH); HÖLAND, Wolfram, CH-9494 Schaan (LI)
(74) Vertreter: Uexküll & Stolberg
(86) Internationale Anmeldenummer: PCT/EP2015/060765
(87) Internationale Veröffentlichungsnummer: WO 2015/173394

(56) Entgegenhaltungen:
- EP-A1- 0 788 093
- EP-A1- 2 930 156
- US-A- 3 804 608
- US-A1- 2003 099 062
- US-A1- 2012 283 086
- US-A1- 2013 149 433

## Beschreibung

Die Erfindung betrifft die Verwendung einer Glaskeramik mit Tiefquarz als Hauptkristallphase sowie von Vorstufen zur Herstellung dieser Glaskeramik als Dentalmaterial und insbesondere zur Herstellung von dentalen Restaurationen.

Glaskeramiken mit quarz-ähnlichen Kristallen sind aus dem Stand der Technik bereits bekannt. Dabei handelt es sich üblicherweise um Glaskeramiken mit sogenannten Hochquarz-Mischkristallen. Diese Kristalle enthalten verschiedenartige Zusatzionen im SiO₂-Gerüstsilikat, die dieser besonderen Kristallart auch bei Raumtemperatur eine metastabile Existenz erlaubt. Wären diese Ionen nicht in der Glaskeramik enthalten, würde der bei hohen Temperaturen in der Glaskeramik gebildete Hochquarz sich bei 573°C in Tiefquarz umwandeln. Höland und Beall beschreiben, dass Glaskeramiken mit Kristallen in Hochquarzstruktur die besondere Eigenschaft der geringen Wärmeausdehnung oder sogar der Nullausdehnung in einem grossen Temperaturbereich besitzen ("Glass-Ceramic Technology" 2. Aufl., Wiley, 2012, 272-273). Üblicherweise werden für derartige Glaskeramiken lineare thermische Ausdehnungskoeffizienten (WAK) von kleiner als 1,5·10⁻⁶ K⁻¹ (im Temperaturbereich von 20 - 700 °C) gemessen. Selbst Glaskeramiken mit einem negativen Ausdehnungskoeffizienten können mit Hilfe der Hochquarzstruktur bereitgestellt werden. Weiter sind aus der EP 916 625 Lithiumdisilikat-Glaskeramiken bekannt, die Lithiumdisilikat als Hauptkristallphase enthalten und aufgrund ihrer hohen Transluzenz und sehr guten mechanischen Eigenschaften besonders im Dentalbereich und dabei vornehmlich zur Herstellung von Kronen und Brücken Anwendung finden.

Der Erfindung liegt die Aufgabe zugrunde, die Verwendung einer Glaskeramik bereit zu stellen, die sich neben einer hohen Festigkeit und sehr guten optischen Eigenschaften auch durch einen hohen Wärmeausdehnungskoeffizienten auszeichnet. Die Glaskeramik soll weiter in einfacher Weise insbesondere durch maschinelle Bearbeitung zu dentalen Restaurationen verarbeitbar sein und sich damit ausgezeichnet als restauratives Dentalmaterial eignen. Dabei wäre es wünschenswert, wenn der Glaskeramik auch mittels Heißpressen die gewünschte Form gegeben werden könnte.

Diese Aufgaben werden durch die Verwendung von Glaskeramik als Dentalmaterial wie in einem der Ansprüche 1 bis 12 und 14 bis 16 definiert gelöst. Gegenstand der Erfindung sind ebenfalls die Verwendung von Ausgangsglas wie in einem der Ansprüche 13 bis 16 definiert und die Verfahren wie in einem der Ansprüche 17 bis 19 definiert.

Die erfindungsgemäß als Dentalmaterial verwendete Glaskeramik zeichnet sich dadurch aus, dass sie die folgenden Komponenten enthält

| Komponente | Gew.-% |
|---|---|
| SiO₂ | 58,0 bis 92,0 |
| Li₂O | 2,0 bis 10,0 |

und Tiefquarz als Hauptkristallphase enthält.

Diese Glaskeramik zeigt überraschenderweise eine vorteilhafte Kombination von für ein restauratives Dentalmaterial wünschenswerten mechanischen und optischen Eigenschaften und sie kann zudem in einer für ein Dentalmaterial wünschenswerten Weise die gewünschte Form gegeben werden.

Die erfindungsgemäß verwendete Glaskeramik enthält insbesondere 60,0 bis 90,0, vorzugsweise 70,0 bis 83,0 Gew.-% und besonders bevorzugt 73,0 bis 81,0 Gew.-% SiO₂.

Es ist weiter bevorzugt, dass die Glaskeramik 2,8 bis 9,0, insbesondere 5,0 bis 9,0 und besonders bevorzugt 5,0 bis 7,8 Gew.-% Li₂O enthält. Li₂O dient der Verbesserung der Erschmelzbarkeit der Ausgangsgläser. Weiter fördert es zudem die Mobilität der Ionen in der Glasmatrix und es wird angenommen, dass sich dies positiv auf die Kristallisation von manchen Kristallphasen, z.B. von Tiefquarz und Lithiumsilikat, auswirkt.

Auch ist es bevorzugt, dass die Glaskeramik neben Li₂O weiteres Alkalimetalloxid Me^{I}₂O in einer Menge 0 bis 13,0, insbesondere 1,0 bis 13,0 und besonders bevorzugt 2,0 bis 13,0 Gew.-% enthält. Der Begriff "weiteres Alkalimetalloxid Me¹₂O" bezeichnet Alkalimetalloxid mit Ausnahme von Li₂O, wobei dieses Me^{I}₂O insbesondere ausgewählt ist aus Na₂O, K₂O, Rb₂O und/oder Cs₂O. Besonders bevorzugt enthält die Glaskeramik mindestens eines und insbesondere alle der folgenden weiteren Alkalimetalloxide Me^{I}₂O in den angegebenen Mengen:

| Komponente | Gew.-% |
|---|---|
| Na₂O | 0 bis 3,0 |
| K₂O | 0 bis 5,0 |
| Rb₂O | 0 bis 7,0 |
| Cs₂O | 0 bis 13,0. |

In einer besonders bevorzugten Ausführungsform enthält die erfindungsgemäß verwendete Glaskeramik 1,0 bis 4,0 Gew.-% K₂O.

Des Weiteren ist es bevorzugt, dass die Glaskeramik 0 bis 11,0 und insbesondere 1,0 bis 7,0 Gew.-% Oxid zweiwertiger Elemente Me^{II}O enthält, wobei dieses Oxid Me^{II}O insbesondere ausgewählt ist aus MgO, CaO, SrO und/oder ZnO. Besonders bevorzugt enthält die Glaskeramik mindestens eines und insbesondere alle der folgenden Oxide zweiwertiger Elemente Me^{II}O in den angegebenen Mengen:

| Komponente | Gew.-% |
|---|---|
| CaO | 0 bis 4,5 |
| MgO | 0 bis 7,0 |
| SrO | 0 bis 5,0 |
| ZnO | 0 bis 4,0. |

In einer besonders bevorzugten Ausführungsform enthält die erfindungsgemäß verwendete Glaskeramik 1,0 bis 7,0, und insbesondere 1,0 bis 2,0 Gew.-% MgO.

Es ist weiter eine Glaskeramik bevorzugt, die 0 bis 10,0 und insbesondere 2,0 bis 9,0 Gew.-% Oxid dreiwertiger Elemente Me^{III}₂O₃ enthält, wobei dieses Me^{III}₂O₃ insbesondere ausgewählt ist aus Al₂O₃, B₂O₃, Y₂O₃, La₂O₃, Ga₂O₃ und/oder In₂O₃. Besonders bevorzugt enthält die Glaskeramik mindestens eines und insbesondere alle der folgenden Oxide dreiwertiger Elemente Me^{III}₂O₃ in den angegebenen Mengen:

| Komponente | Gew.-% |
|---|---|
| Al₂O₃ | 0 bis 8,0 |
| Y₂O₃ | 0 bis 3,0 |
| B₂O₃ | 0 bis 5,0 |
| Ga₂O₃ | 0 bis 2,0 |
| In₂O₃ | 0 bis 1,0 |
| La₂O₃ | 0 bis 2,0. |

In einer besonders bevorzugten Ausführungsform enthält die erfindungsgemäß verwendete Glaskeramik 2,0 bis 8,0 Gew.-% Al₂O₃.

Ferner ist eine Glaskeramik bevorzugt, die weiteres Oxid vierwertiger Elemente Me^{IV}O₂ in einer Menge 0 bis 21,0 Gew.-% enthält. Der Begriff "weiteres Oxid vierwertiger Elemente Me^{IV}O₂" bezeichnet vierwertige Oxide mit Ausnahme von SiO₂, wobei dieses Me^{IV}O₂ insbesondere ausgewählt ist aus ZrO₂, GeO₂, CeO₂, TiO₂ und/oder SnO₂. Besonders bevorzugt enthält die Glaskeramik mindestens eines und insbesondere alle der folgenden weiteren Oxide vierwertiger Elemente Me^{IV}O₂ in den angegebenen Mengen:

| Komponente | Gew.-% |
|---|---|
| ZrO₂ | 0 bis 11,0 |
| TiO₂ | 0 bis 5,0 |
| SnO₂ | 0 bis 3,0 |
| GeO₂ | 0 bis 21,0 |
| CeO₂ | 0 bis 3,0. |

In einer weiteren bevorzugten Ausführungsform enthält die Glaskeramik 0 bis 7,0, insbesondere 0 bis 6,5, besonders bevorzugt 1,0 bis 6,5 und ganz besonders bevorzugt 2,0 bis 6,5 Gew.-% P₂O₅.

P₂O₅ kann als Keimbildner fungieren. Das Vorhandensein eines Keimbildners ist für die Bildung von Tiefquarz als Hauptkristallphase jedoch nicht zwingend erforderlich.

Außerdem ist eine Glaskeramik bevorzugt, die weiteres Oxid fünfwertiger Elemente Me^{V}₂O₅ in einer Menge 0 bis 6,0 und insbesondere 0 bis 5,0 Gew.-% enthält. Der Begriff "weiteres Oxid fünfwertiger Elemente Me^{V}₂O₅" bezeichnet fünfwertige Oxide mit Ausnahme von P₂O₅, wobei dieses Me^{V}₂O₅ insbesondere ausgewählt ist aus V₂O₅, Ta₂O₅ und/oder Nb₂O₅. Besonders bevorzugt enthält die Glaskeramik mindestens eines und insbesondere alle der folgenden weiteren Oxide fünfwertiger Elemente Me^{V}₂O₅ in den angegebenen Mengen:

| Komponente | Gew.-% |
|---|---|
| V₂O₅ | 0 bis 6,0 |
| Ta₂O₅ | 0 bis 5,0 |
| Nb₂O₅ | 0 bis 5,0. |

Auch ist eine Glaskeramik bevorzugt, die 0 bis 6,0 Gew.-% Oxid sechswertiger Elemente Me^{VI}O₃ enthält, wobei dieses Me^{VI}O₃ insbesondere ausgewählt ist aus WO₃ und/oder MoO₃. Besonders bevorzugt enthält die Glaskeramik mindestens eines und insbesondere alle der folgenden Oxide Me^{VI}O₃ in den angegebenen Mengen:

| Komponente | Gew.-% |
|---|---|
| WO₃ | 0 bis 6,0 |
| MoO₃ | 0 bis 5,0. |

Weiterhin ist eine Glaskeramik bevorzugt, die 0 bis 5,0 und insbesondere 0 bis 1,0 Gew.-% Fluor enthält.

Besonders bevorzugt ist eine Glaskeramik, die mindestens eine und bevorzugt alle der folgenden Komponenten in den angegebenen Mengen enthält:

| Komponente | Gew.-% |
|---|---|
| SiO₂ | 58,0 bis 92,0 |
| Li₂O | 2,0 bis 10,0 |
| Me^{I}₂O | 0 bis 13,0 |
| Me^{II}O | 0 bis 11,0 |
| Me^{III}₂O₃ | 0 bis 10,0 |
| Me^{IV}O₂ | 0 bis 21,0 |
| P₂O₅ | 0 bis 7,0 |
| Me^{V}₂O₅ | 0 bis 6,0 |
| Me^{VI}O₃ | 0 bis 6,0 |
| Fluor | 0 bis 5,0, |

wobei Me^{I}₂O, Me^{II}O, Me^{III}₂O₃, Me^{IV}O₂, Me^{V}₂O₅ und Me^{VI}O₃ die oben angegebene Bedeutung haben.

In einer weiteren besonders bevorzugten Ausführungsform enthält die Glaskeramik mindestens eine und bevorzugt alle der folgenden Komponenten:

| Komponente | Gew.-% |
|---|---|
| SiO₂ | 60,0 bis 90,0 |
| Li₂O | 2,8 bis 9,0 |
| Na₂O | 0 bis 3,0 |
| K₂O | 0 bis 5,0 |
| Rb₂O | 0 bis 7,0 |
| Cs₂O | 0 bis 13,0 |
| CaO | 0 bis 4,5 |
| MgO | 0 bis 7,0 |
| SrO | 0 bis 5,0 |
| ZnO | 0 bis 4,0 |
| Al₂O₃ | 0 bis 8,0 |
| Y₂O₃ | 0 bis 3,0 |
| B₂O₃ | 0 bis 5,0 |
| Ga₂O₃ | 0 bis 2,0 |
| In₂O₃ | 0 bis 1,0 |
| La₂O₃ | 0 bis 2,0 |
| ZrO₂ | 0 bis 11,0 |
| TiO₂ | 0 bis 5,0 |
| SnO₂ | 0 bis 3,0 |
| GeO₂ | 0 bis 21,0 |
| CeO₂ | 0 bis 3,0 |
| P₂O₅ | 0 bis 6,5 |
| Ta₂O₅ | 0 bis 5,0 |
| Nb₂O | 0 bis 5,0 |
| V₂O₅ | 0 bis 6,0 |
| WO₃ | 0 bis 6,0 |
| MoO₃ | 0 bis 5,0 |
| Fluor | 0 bis 1,0. |

Manche der vorstehend genannten Komponenten können als Färbemittel und/oder Fluoreszenzmittel dienen. Die erfindungsgemäß verwendete Glaskeramik kann darüber hinaus noch weitere Färbemittel und/oder Fluoreszenzmittel enthalten, die insbesondere aus anorganischen Pigmente und/oder Oxiden von d- und f-Elementen, wie z.B. den Oxiden von Sc, Mn, Fe, Co, Pr, Nd, Tb, Er, Dy, Gd, Eu und Yb, ausgewählt sein können. Als weitere Färbemittel können auch Metallkolloide, z.B. von Ag, Au und Pd, verwendet werden, die zusätzlich auch als Keimbildner fungieren können. Diese Metallkolloide können z.B. durch Reduktion von entsprechenden Oxiden, Chloriden oder Nitraten während der Schmelz- und Kristallisationsprozesse gebildet werden.

Die Eigenschaften der Glaskeramik werden maßgeblich durch die Hauptkristallphase beeinflusst. Die erfindungsgemäß verwendete Glaskeramik enthält Tiefquarz als Hauptkristallphase.

Mit dem Begriff "Hauptkristallphase" wird die Kristallphase bezeichnet, die von allen in der Glaskeramik vorhandenen Kristallphasen den höchsten Massenanteil hat. Die Bestimmung der Massen der Kristallphasen erfolgt dabei insbesondere mit der Rietveld-Methode. Ein geeignetes Verfahren zur quantitativen Analyse der Kristallphasen mittels der Rietveld-Methode ist z.B. in der Dissertation von M. Dittmer "Gläser und Glaskeramiken im System MgO-Al2O3-SiO2 mit ZrO2 als Keimbildner", Universität Jena 2011, beschrieben.

Es ist weiter bevorzugt, dass die erfindungsgemäß verwendete Glaskeramik 5,0 bis 50,0 und insbesondere 10,0 bis 30,0 Gew.-% Tiefquarz als Kristallphase enthält.

Die erfindungsgemäß verwendete Glaskeramik kann zusätzlich zu Tiefquarz als Hauptkristallphase noch weitere Kristallphasen, wie insbesondere Lithiumphosphat und/oder Lithiumsilikat enthalten. Ebenso können in der erfindungsgemäß verwendeten Glaskeramik auch noch weitere nanoskalige Phasen in amorpher oder kristalliner Form vorhanden sein.

Es ist bevorzugt, dass die erfindungsgemäß verwendete Glaskeramik 5,0 bis 30,0 und insbesondere 10,0 bis 25,0 Gew.-% Lithiumdisilikat enthält.

Die Art und die Menge der gebildeten Kristallphasen können insbesondere durch die Zusammensetzung des Ausgangsglases sowie die Wärmebehandlung gesteuert werden, die zur Herstellung der Glaskeramik aus dem Ausgangsglas angewendet wird. Die Beispiele veranschaulichen dies anhand der Variation der Zusammensetzung und der angewendeten Wärmebehandlung.

Die erfindungsgemäß verwendete Glaskeramik weist einen thermischen Ausdehnungskoeffizienten WAK (gemessen im Bereich von 100 bis 500°C) von insbesondere mindestens 5,0·10⁻⁶ K⁻¹, bevorzugt 10,0 bis 20,0·10⁻⁶ K⁻¹ und besonders bevorzugt 13,0 bis 18,0·10⁻⁶ K⁻¹ auf. Der WAK wird gemäß ISO 6872 (2008) bestimmt. Die erfindungsgemäß verwendete Glaskeramik zeichnet sich durch eine sehr gute chemische Beständigkeit aus. Zur Bestimmung der chemischen Beständigkeit wurde die Glaskeramik gemäß ISO-Norm 6872 (2008) geprüft, indem der Masseverlust bei Lagerung in wässriger Essigsäure bestimmt wurde. Die erfindungsgemäß verwendete Glaskeramik zeigte dabei einen Masseverlust von vorzugsweise weniger als 30 µg/cm².

Ferner zeichnet sich die erfindungsgemäß verwendete Glaskeramik insbesondere durch mechanische Eigenschaften aus, die eine besonders einfache und schnelle maschinelle Bearbeitung gestatten, um die Glaskeramik z.B. in die Form einer Dentalrestauration zu bringen.

Die Glaskeramik weist eine biaxiale Bruchfestigkeit von vorzugsweise mindestens 200 MPa und besonders bevorzugt 200 bis 500 MPa auf. Die biaxiale Bruchfestigkeit wurde gemäß ISO 6872 (2008) (Kolben-auf-drei-Kugeln-Prüfung) bestimmt.

Die Transluzenz der Glaskeramik wurde in Form des Kontrastwerts (CR-Wert) gemäß British Standard BS 5612 bestimmt und er betrug vorzugsweise 35 bis 80.

Die Erfindung betrifft ebenfalls die Verwendung von Vorstufen mit entsprechender Zusammensetzung, aus denen die Glaskeramik durch Wärmebehandlung hergestellt werden kann. Diese Vorstufen sind ein entsprechend zusammengesetztes Ausgangsglas und ein entsprechend zusammengesetztes Ausgangsglas mit Keimen. Die Bezeichnung "entsprechender Zusammensetzung" bedeutet, dass diese Vorstufen die gleichen Komponenten in den gleichen Mengen wie die Glaskeramik enthalten, wobei die Komponenten mit Ausnahme von Fluor als Oxide berechnet werden, so wie es bei Gläsern und Glaskeramiken üblich ist.

Die Erfindung betrifft daher ebenfalls die Verwendung eines Ausgangsglases, das die Komponenten der erfindungsgemäß verwendeten Glaskeramik enthält, als Dentalmaterial.

Das erfindungsgemäß verwendete Ausgangsglas enthält daher als Komponenten

| Komponente | Gew.-% |
|---|---|
| SiO₂ | 58,0 bis 92,0 |
| Li₂O | 2,0 bis 10,0. |

Weiter kann das Ausgangsglas auch noch andere Komponenten enthalten, wie sie oben für die erfindungsgemäß verwendete Glaskeramik angegeben sind. Es sind alle solche Ausführungsformen für die Komponenten des Ausgangsglases bevorzugt, die auch für die Komponenten der erfindungsgemäß verwendeten Glaskeramik als bevorzugt angegeben sind.

Die Erfindung betrifft ebenfalls die Verwendung eines Ausgangsglases, das Keime für die Kristallisation von Tiefquarz enthält, als Dentalmaterial.

Durch Wärmebehandlung des Ausgangsglases kann zunächst das Ausgangsglas mit Keimen erzeugt werden, welches seinerseits durch weitere Wärmebehandlung in die Glaskeramik mit Tiefquarz als Hauptkristallphase umgewandelt werden kann.

Die Herstellung des Ausgangsglases erfolgt insbesondere in der Weise, dass eine Mischung von geeigneten Ausgangsmaterialien, wie z.B. Carbonaten, Oxiden, und Phosphaten, bei Temperaturen von insbesondere 1500 bis 1800°C für 0,5 bis 10 h erschmolzen wird. Zur Erzielung einer besonders hohen Homogenität wird die erhaltene Glasschmelze in Wasser gegossen, um ein Glasgranulat zu bilden, und das erhaltene Granulat wird dann erneut aufgeschmolzen.

Die Schmelze kann dann in Formen, z.B. Stahl- oder Graphitformen, gegossen werden, um Rohlinge des Ausgangsglases, sogenannte Massivglasrohlinge oder monolithische Rohlinge, zu erzeugen. Üblicherweise werden diese monolithischen Rohlinge zunächst entspannt, z.B. indem sie 5 bis 120 min bei 450 bis 600°C gehalten werden. Diese Entspannung in dem angegebenen Temperaturbereich führt in der Regel zur Bildung von Keimen für die Kristallisation von Tiefquarz-Kristallphase.

Es ist ebenfalls möglich, die Schmelze erneut in Wasser zu geben, um eine Fritte, d.h. ein Granulat, herzustellen. Diese Fritte kann nach Mahlen und gegebenenfalls Zugabe weiterer Komponenten, wie Bindemitteln und/oder Färbe- und Fluoreszenzmitteln, zu einem Rohling, einem sogenannten Pulverpressling, gepresst werden.

Schließlich kann das Ausgangsglas nach der Erzeugung einer Glasfritte auch zu einem Pulver verarbeitet werden.

Aus dem Ausgangsglas kann dann durch Wärmebehandlung das Ausgangsglas mit Keimen erzeugt werden. Dies wird auch als Keimbildungsprozess bezeichnet.

Offenbart wird daher ebenfalls ein Verfahren zur Herstellung des Ausgangsglases mit Keimen für die

Kristallisation von Tiefquarz, bei dem das Ausgangsglas einer Wärmebehandlung bei einer Temperatur von 450 bis 600°C und insbesondere 500 bis 550°C für eine Dauer von insbesondere 5 bis 120 min und vorzugsweise 10 bis 40 min unterworfen wird.

Aus dem Ausgangsglas mit Keimen kann dann durch Wärmebehandlung die erfindungsgemäß verwendete Glaskeramik gebildet werden.

Offenbart wird daher ebenfalls ein Verfahren zur Herstellung der erfindungsgemäß verwendeten Glaskeramik, bei dem das Ausgangsglas, insbesondere das Ausgangsglas mit Keimen, mindestens einer Wärmebehandlung bei einer Temperatur von 700 bis 950°C für eine Dauer von insbesondere 5 bis 40 min und vorzugsweise 10 bis 30 min unterworfen wird.

Das Ausgangsglas oder das Ausgangsglas mit Keimen kann z.B. in Form eines Massivglasrohlings oder eines Pulverpresslings der mindestens einen Wärmebehandlung unterzogen werden.

Die im obigen Verfahren durchgeführte mindestens eine Wärmebehandlung kann auch im Rahmen eines Heißpressens, insbesondere eines Massivglasrohlings, oder im Rahmen eines Aufsinterns, insbesondere eines Pulvers, erfolgen.

Somit wird in einer bevorzugten Ausführungsform ein Verfahren zur Herstellung der erfindungsgemäß verwendeten Glaskeramik offenbart, bei dem
(a) Pulver des Ausgangsglases, gegebenenfalls nach Zugabe weiterer Komponenten, wie z.B. Presshilfsmittel, Färbemittel und/oder Fluoreszenzmittel, zu einem Pulverpressling gepresst wird, und
(b) der Pulverpressling einer Wärmebehandlung bei einer Temperatur von 700 bis 950°C für eine Dauer von insbesondere 5 bis 40 min unterworfen wird.

In einer weiteren bevorzugten Ausführungsform wird ein Verfahren zur Herstellung der erfindungsgemäß verwendeten Glaskeramik offenbart, bei dem
(a') Schmelze des Ausgangsglases zu einem Glasrohling geformt wird, insbesondere durch Giessen in eine Form, und
(b') der Glasrohling einer Wärmebehandlung bei einer Temperatur von 700 bis 900°C für eine Dauer von insbesondere 5 bis 40 min unterworfen wird.

In beiden bevorzugten Ausführungsformen des Verfahrens kann vor der Wärmebehandlung in Schritt (b) oder (b') eine weiter oben beschriebene Keimbildung vorgenommen werden.

Die erfindungsgemäß verwendeten Glaskeramiken und die erfindungsgemäß verwendeten Gläser liegen insbesondere in Form von Pulvern, Granulaten oder Rohlingen in beliebiger Form und Größe, z.B. monolithischen Rohlingen, wie Plättchen, Quadern oder Zylindern, oder Pulverpresslingen, in ungesinterter, teilgesinterter oder dichtgesinterter Form, vor. In diesen Formen können sie einfach weiterverarbeitet werden, z.B. zu dentalen Restaurationen. Sie können aber auch in Form von dentalen Restaurationen, wie Inlays, Onlays, Kronen, Veneers, Schalen oder Abutments, vorliegen.

Aus den erfindungsgemäß verwendeten Glaskeramiken und den erfindungsgemäß verwendeten Gläsern können dentale Restaurationen, wie Brücken, Inlays, Onlays, Kronen, Veneers, Schalen oder Abutments, hergestellt werden. Die Erfindung betrifft daher deren Verwendung als Dentalmaterial und insbesondere deren Verwendung zur Herstellung dentaler Restaurationen. Dabei ist es bevorzugt, dass der Glaskeramik oder dem Glas durch Verpressen oder maschinelle Bearbeitung die Form der gewünschten dentalen Restauration gegeben wird.

Das Verpressen erfolgt üblicherweise unter erhöhtem Druck und erhöhter Temperatur. Es ist bevorzugt, dass das Verpressen bei einer Temperatur von 700 bis 1200°C erfolgt. Weiter ist es bevorzugt, das Verpressen bei einem Druck von 10 bis 30 bar durchzuführen. Beim Verpressen wird durch viskoses Fließen des eingesetzten Materials die gewünschte Formänderung erreicht. Es können für das Verpressen das Ausgangsglas und das Ausgangsglas mit Keimen sowie bevorzugt die Glaskeramik verwendet werden. Dabei können die Gläser und Glaskeramiken insbesondere in Form von Rohlingen in beliebiger Form und Größe, z.B. Massivrohlingen oder Pulverpresslingen, z.B. in ungesinterter, teilgesinterter oder dichtgesinterter Form, eingesetzt werden.

Die maschinelle Bearbeitung erfolgt üblicherweise durch materialabtragende Verfahren und insbesondere durch Fräsen und/oder Schleifen. Es ist besonders bevorzugt, dass die maschinelle Bearbeitung im Rahmen eines CAD/CAM-Verfahrens durchgeführt wird. Für die maschinelle Bearbeitung können das Ausgangsglas, das Ausgangsglas mit Keimen sowie die Glaskeramik verwendet werden. Dabei können die Gläser und Glaskeramiken insbesondere in Form von Rohlingen, z.B. Massivrohlingen oder Pulverpresslingen, z.B. in ungesinterter, teilgesinterter oder dichtgesinterter Form, eingesetzt werden. Für die maschinelle Bearbeitung wird bevorzugt die Glaskeramik verwendet. Die Glaskeramik kann auch in einer noch nicht vollständig kristallisierten Form eingesetzt werden, die durch Wärmebehandlung bei niedrigerer Temperatur erzeugt wurde. Dies bietet den Vorteil, dass eine leichtere maschinelle Bearbeitung und damit der Einsatz von einfacheren Apparaten zur maschinellen Bearbeitung möglich ist. Nach der maschinellen Bearbeitung eines solchen teilkristallisierten Materials wird dieses regelmäßig einer weiteren Wärmebehandlung unterzogen, um eine weitere Kristallisation von Tiefquarz als Kristallphase hervorzurufen.

Die erfindungsgemäß verwendeten Glaskeramiken und die erfindungsgemäß verwendeten Gläser eignen sich allerdings auch als Beschichtungsmaterial von z.B. Keramiken, Glaskeramiken und Metallen. Die Erfindung ist daher ebenfalls auf die Verwendung der Gläser oder Glaskeramiken zur Beschichtung von insbesondere Keramiken, Glaskeramiken und Metallen gerichtet.

Die Erfindung betrifft auch ein Verfahren zur Beschichtung von Keramiken, Glaskeramiken und Metallen, bei dem erfindungsgemäß verwendete Glaskeramik oder erfindungsgemäß verwendetes Glas auf die Keramik, die Glaskeramik oder das Metall aufgebracht und einer Temperatur von mindestens 600°C ausgesetzt wird.

Dies kann insbesondere durch Aufsintern und bevorzugt durch Aufpressen erfolgen. Beim Aufsintern wird die Glaskeramik oder das Glas in üblicher Weise, z.B. als Pulver, auf das zu beschichtende Material, wie Keramik, Glaskeramik oder Metall, aufgebracht und anschließend gesintert. Bei dem bevorzugten Aufpressen wird erfindungsgemäß verwendete Glaskeramik oder erfindungsgemäß verwendetes Glas, z.B. in Form von Pulverpresslingen oder monolithischen Rohlingen, bei einer erhöhten Temperatur, von z.B. 700 bis 1200°C, und unter Anwendung von Druck, z.B. 10 bis 30 bar, aufgepresst. Hierzu können insbesondere die in der EP 231 773 beschriebenen Verfahren und der dort offenbarte Pressofen eingesetzt werden. Geeignete kommerzielle Öfen sind die Öfen vom Typ Programat von Ivoclar Vivadent AG, Liechtenstein.

Aufgrund der vorstehend geschilderten Eigenschaften der erfindungsgemäß verwendeten Glaskeramiken und der erfindungsgemäß verwendeten Gläser eignen sich diese insbesondere zum Einsatz in der Zahnheilkunde. Gegenstand der Erfindung ist daher die Verwendung der Glaskeramiken oder Gläser als Dentalmaterial und insbesondere zur Herstellung dentaler Restaurationen oder als Beschichtungsmaterial für dentale Restaurationen, wie Kronen, Brücken und Abutments.

Die Erfindung betrifft mithin auch ein Verfahren zur Herstellung einer dentalen Restauration, insbesondere Brücke, Inlay, Onlay, Veneer, Abutment, Teilkrone, Krone oder Schale, bei dem der erfindungsgemäß verwendeten Glaskeramik oder dem erfindungsgemäß verwendeten Ausgangsglas durch Verpressen, Sintern oder maschinelle Bearbeitung, insbesondere im Rahmen eines CAD/CAM-Verfahrens, die Form der gewünschten dentalen Restauration gegeben wird.

Die Erfindung wird im Folgenden anhand von sie nichtbeschränkenden Beispielen näher erläutert.

### Beispiele

### Beispiele 1 bis 24 - Zusammensetzung und Kristallphasen

Es wurden insgesamt 24 Gläser und Glaskeramiken mit der aus Tabelle I angegebenen Zusammensetzung über Erschmelzung entsprechender Ausgangsgläser, ggf. Keimbildung oder Entspannung, und anschließende Wärmebehandlung zur Kristallisation hergestellt.

Dabei bedeuten in Tabelle 1:
- T_{g}: Glasübergangstemperatur, bestimmt mittels DSC
- T_{S} und t_{S}: Angewendete Temperatur und Zeit für Erschmelzung des Ausgangsglases
- T_{Kb} und t_{Kb}: Angewendete Temperatur und Zeit für Keimbildung bzw. Entspannung des Ausgangsglases
- T_{C} und t_{C}: Angewendete Temperatur und Zeit für Kristallisation von Massivglasblöcken
- T_{Sinter} und t_{Sinter}: Angewendete Temperatur und Zeit für Kristallisation von Pulverpresslingen
- T_{Press} und t_{Press}: Angewendete Temperatur und Zeit für Kristallisation von Massivglasblöcken durch Heißpressen
- CR-Wert: Kontrastwerts der Glaskeramik gemäß British Standard BS 5612 bestimmt unter Verwendung von: Gerät: Spektrometer CM-3700d (Konica-Minolta) Messparameter: Messfläche: 7mm x 5 mm Messart: Remission / Reflektion Messbereich: 400 nm -700 nm Probengröße: Durchmesser: 15-20 mm Dicke: 2 mm +- 0.025 mm Planparallelität: +- 0.05 mm Oberflächenrauhigkeit : ca. 18 µm.
- WAK: Thermischer Ausdehnungskoeffizient der Glaskeramik gemäß ISO 6872 (2008), gemessen im Bereich von 100 bis 500°C)

In den Beispielen 1 bis 24 wurden zunächst die Ausgangsgläser im 100 bis 200 g Maßstab aus üblichen Rohstoffen bei der Temperatur T_{S} für eine Dauer t_{S} erschmolzen. Durch Eingießen der erschmolzenen Ausgangsgläser in Wasser wurden Glasfritten hergestellt. Für die Weiterverarbeitung der Glasfritten wurden die nachstehend angegebenen drei Verfahrensvarianten A), B) und C) benutzt:

### A) Massivglasblöcke

In Beispielen, für die in der Tabelle 1 T_{C} und t_{C} angegeben sind (Beispiele 3-5, 7-12, 14, 16-18 und 20-24), wurden die Glaskeramiken aus Massivglasblöcken hergestellt. Dazu wurden die erhaltenen Glasfritten erneut bei der Temperatur T_{S} für eine Dauer t_{S} aufgeschmolzen. Die erhaltenen Schmelzen des Ausgangsglases wurden anschließend in eine Graphitform gegossen, um Massivglasblöcke zu erzeugen. Diese Glasmonolithe wurden daraufhin in der Regel bei der Temperatur T_{Kb} für eine Dauer t_{Kb} entspannt, wodurch Keimbildung stattfinden konnte. Die keimhaltigen Ausgangsgläser wurden dann für eine Dauer t_{C} auf eine Temperatur T_{C} erwärmt. Dadurch wurden erfindungsgemäß verwendete Glaskeramiken mit Tiefquarz als Hauptkristallphase gebildet, wie durch Röntgenbeugungsuntersuchungen bei Raumtemperatur festgestellt werden konnte.

Es wird angenommen, dass bei dieser Verfahrensvariante eine Volumenkristallisation der Tiefquarz-Kristallphase stattgefunden hat.

### B) Pulverpresslinge

In Beispielen, für die in der Tabelle 1 T_{Sinter} und tsinter angegeben sind (1, 2, 6, 15 und 19), wurden die Glaskeramiken aus Pulverpresslingen hergestellt. Dazu wurden die erhaltenen Glassfritten in einer Zirkonoxidmühle auf eine Korngrösse von < 90µm aufgemahlen. Ca. 4g dieser Pulver wurden anschliessend zu zylinderförmigen Rohlingen verpresst und in einem Sinterofen (Programat^{®} von Ivoclar Vivadent AG) bei einer Temperatur T_{Sinter} und einer Haltezeit von t_{Sinter} zu dichten glaskeramischen Körper gesintert. Durch die Sinterung wurden Glaskeramiken mit Tiefquarz als Hauptkristallphase gebildet, wie durch Röntgenbeugungsuntersuchungen bei Raumtemperatur festgestellt werden konnte.

Es wird angenommen, dass bei dieser Verfahrensvariante eine Oberflächenkristallisation der Tiefquarz-Kristallphase stattgefunden hat.

### C) Heißpressen von Massivglasblöcken

In Beispiel 13, für das T_{Press} und t_{Press} angegeben sind, wurde die Glaskeramik durch Heißpressen von Massivglasblöcken hergestellt.

Dazu wurde die erhaltene Glasfritte erneut bei der Temperatur T_{S} für eine Dauer t_{S} aufgeschmolzen. Die erhaltene Schmelze des Ausgangsglases wurde anschließend in eine vorgewärmte Stahlform gegossen, um Stäbe zu erzeugen. Diese monolithischen Glasstäbe wurden daraufhin bei einer Temperatur T_{Kb} für eine Dauer t_{Kb} entspannt, wodurch Keimbildung stattfinden konnte. Die Stäbe wurden dann in Blöcke einer Masse von ungefähr 4 bis 6 g zersägt. Diese Massivglasblöcke wurden dann in einem Heißpressofen bei der Temperatur T_{Press} und einer Haltezeit von tₚᵣₑₛₛ zu einem Formkörper verpresst. Durch das Heißpressen wurde Glaskeramik mit Tiefquarz als Hauptkristallphase gebildet, wie durch Röntgenbeugungsuntersuchungen des gebildeten Formkörpers bei Raumtemperatur festgestellt werden konnte.

Die gemäß den Beispielen 1 bis 12 und 14 bis 24 erzeugten Glaskeramikblöcke wurden in einer CAD/CAM-Einheit maschinell zu gewünschten Prüfkörpern gemäß Dentalnorm und zu Dentalrestaurationen, wie Kronen, verarbeitet. Dazu wurden die kristallisierten Blöcke mit einem geeigneten Halter versehen und anschließend wurde ihnen in einer Schleifeinheit inLab MC XL der Firma Sirona Dental GmbH, Österreich, die gewünschte Form gegeben.

Für die Glaskeramik gemäß Beispiel 1 wurden zusätzlich die Farbwerte (L,a,b) gemäß DIN5033 und DIN6174 wie folgt bestimmt:

| | |
|---|---|
| L: | 90,68 |
| a: | -0,54 |
| b: | 4,82 |

Die Untersuchung der chemischen Beständigkeit nach ISO 6872 (2008) der Glaskeramik gemäß Beispiel 1 ergab eine Säurelöslichkeit von nur 5 µg/cm².

Weiter wurden gemäß Beispiel 1 erhaltene Glaskeramikblöcke mit passenden Haltern versehen und aus ihnen wurden mit einer inLab-Schleifeinheit der Firma Sirona Dental GmbH Prüfkörper zur Bestimmung der Biaxialfestigkeit herausgeschliffen. Die Prüfkörper wurden auf 15 µm poliert und anschließend wurde ohne weitere thermische Behandlung die Biaxialfestigkeit bestimmt. Die mittlere Festigkeit der so hergestellten Prüfkörper betrug 247 MPa.

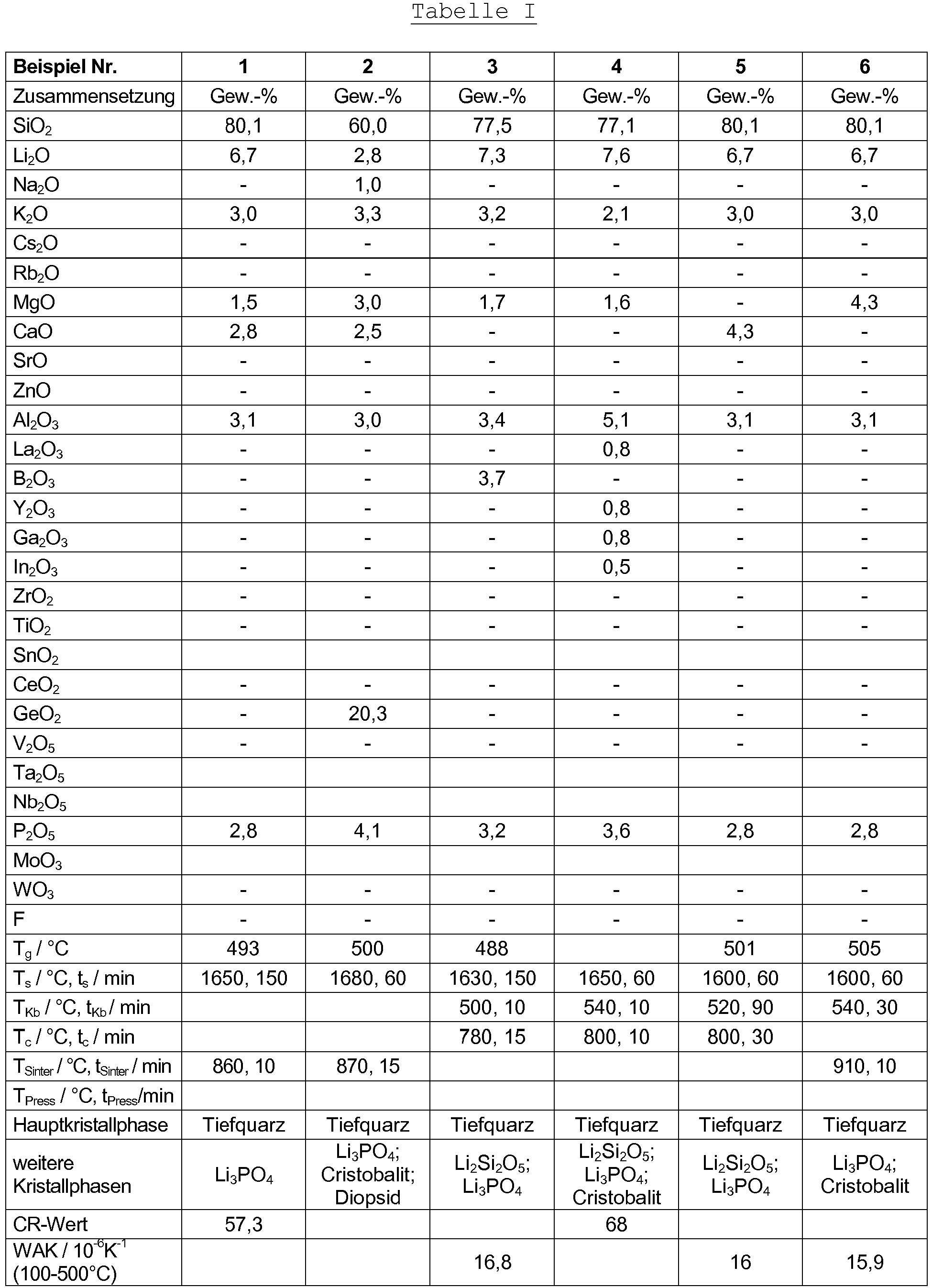

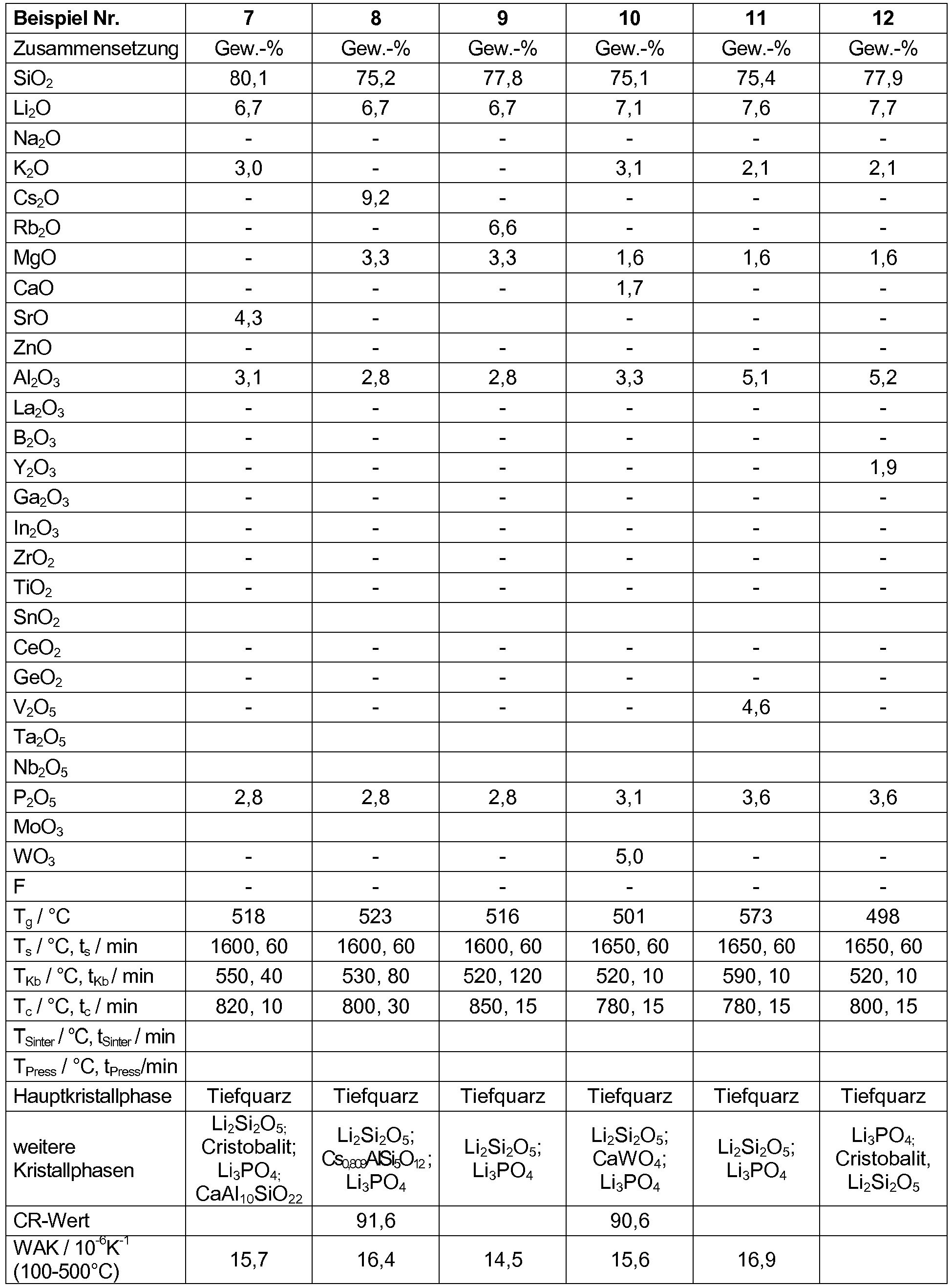

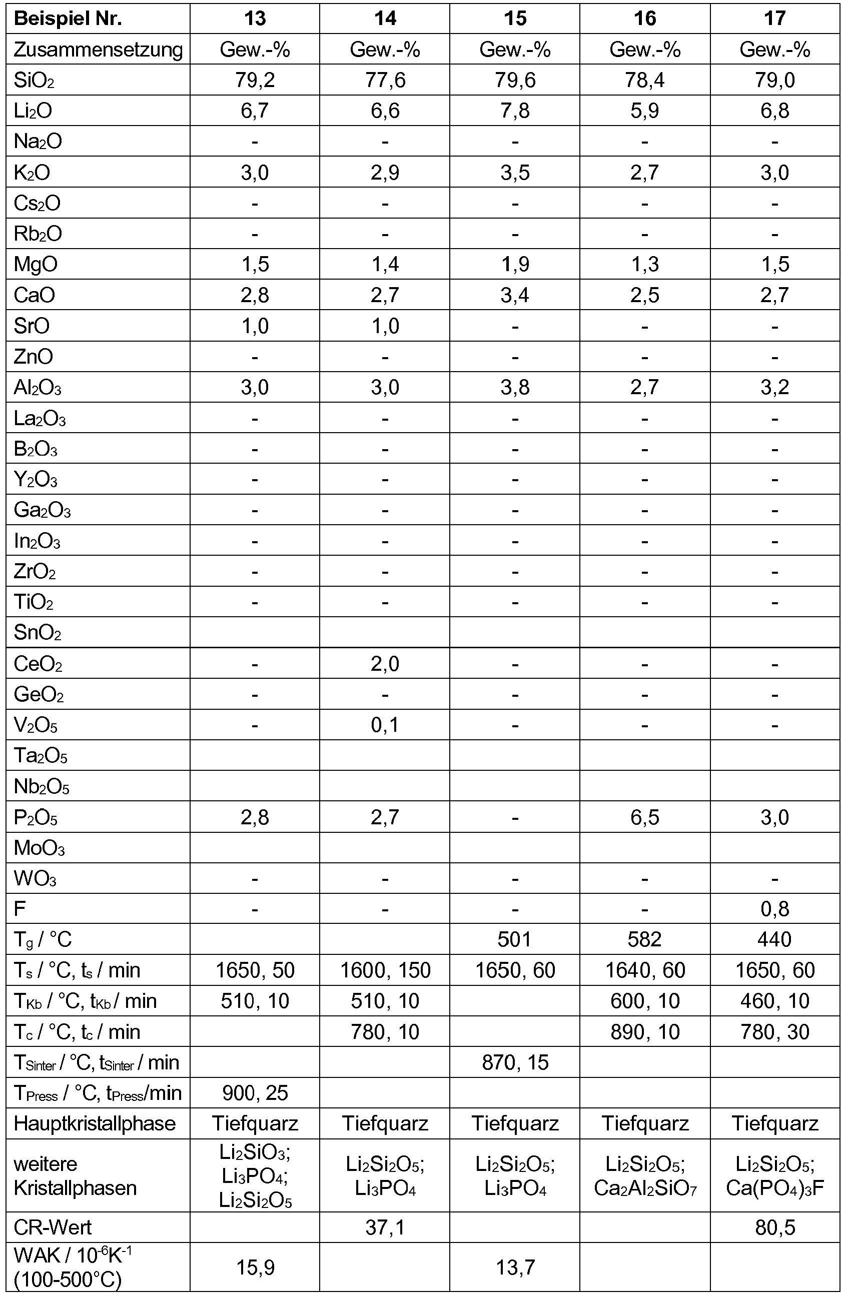

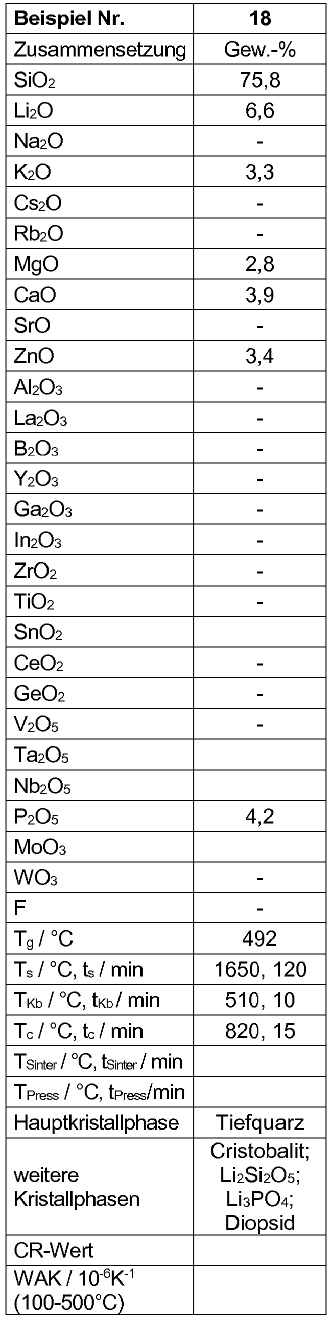

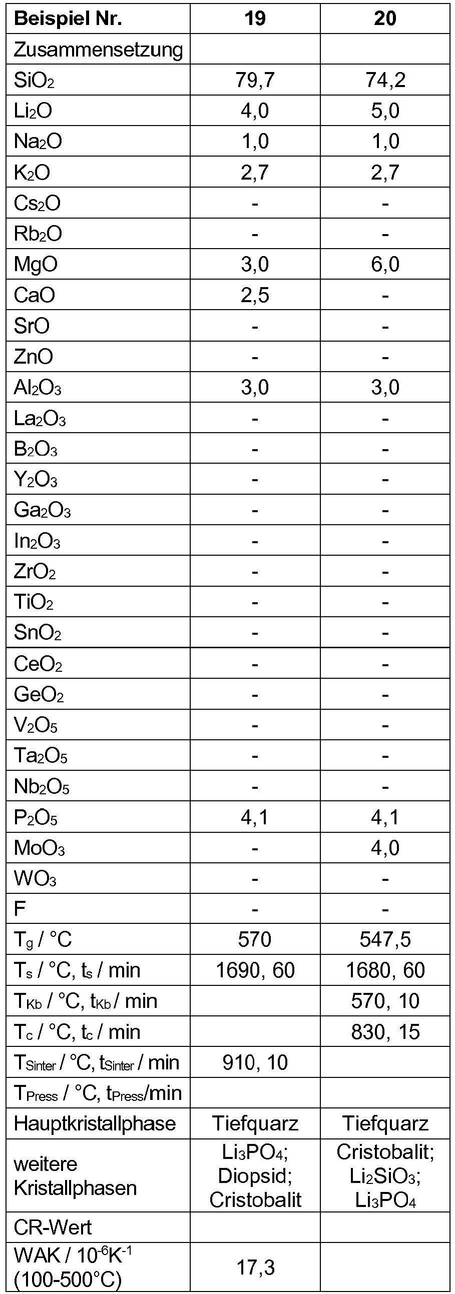

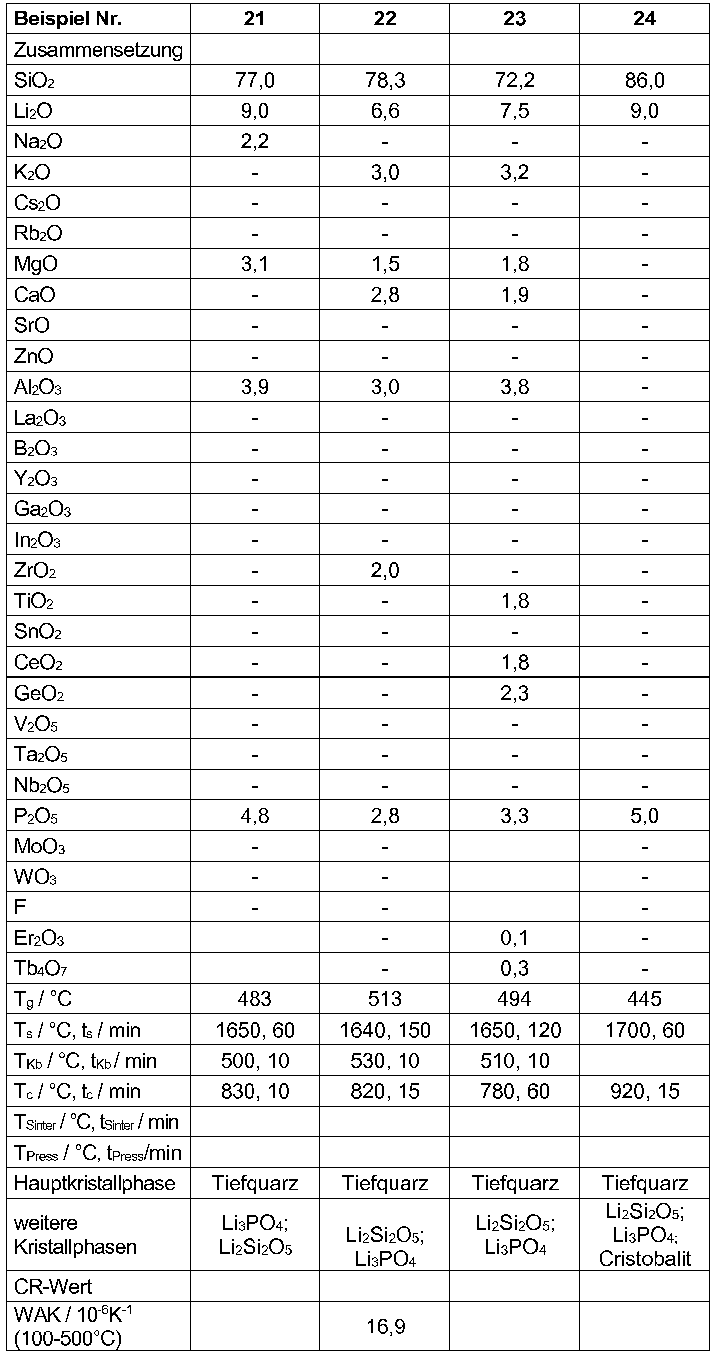

## Patentansprüche

1. Verwendung von Glaskeramik, die die folgenden Komponenten enthält
| Komponente | Gew.-% |
|---|---|
| SiO₂ | 58,0 bis 92,0 |
| Li₂O | 2,0 bis 10,0 |
und Tiefquarz als Hauptkristallphase enthält,
als Dentalmaterial.

2. Verwendung nach Anspruch 1, bei der die Glaskeramik 60,0 bis 90,0, bevorzugt 70,0 bis 83,0 und besonders bevorzugt 73,0 bis 81,0 Gew.-% SiO₂ enthält.

3. Verwendung nach Anspruch 1 oder 2, bei der die Glaskeramik 2,8 bis 9,0, bevorzugt 5,0 bis 9,0 und besonders bevorzugt 5,0 bis 7,8 Gew.% Li₂O enthält.

4. Verwendung nach einem der Ansprüche 1 bis 3, bei der die Glaskeramik 0 bis 13,0, bevorzugt 1,0 bis 13,0 und besonders bevorzugt 2,0 bis 13,0 Gew.-% weiteres Alkalimetalloxid Me^{I}₂O enthält, wobei Me^{I}₂O insbesondere ausgewählt ist aus Na₂O, K₂O, Rb₂O und/oder Cs₂O.

5. Verwendung nach einem der Ansprüche 1 bis 4, bei der die Glaskeramik 0 bis 11,0 und bevorzugt 1,0 bis 7,0 Gew.-% Oxid zweiwertiger Elemente Me^{II}O enthält, wobei Me^{II}O insbesondere ausgewählt ist aus MgO, CaO, SrO und/oder ZnO.

6. Verwendung nach einem der Ansprüche 1 bis 5, bei der die Glaskeramik 0 bis 10,0 und bevorzugt 2,0 bis 9,0 Gew.-% Oxid dreiwertiger Elemente Me^{III}₂O₃ enthält, wobei Me^{III}₂O₃ insbesondere ausgewählt ist aus Al₂O₃, B₂O₃, Y₂O₃, La₂O₃, Ga₂O₃ und/oder In₂O₃.

7. Verwendung nach einem der Ansprüche 1 bis 6, bei der die Glaskeramik 0 bis 21,0 Gew.-% weiteres Oxid vierwertiger Elemente Me^{IV}O₂ enthält, wobei Me^{IV}O₂ insbesondere ausgewählt ist aus ZrO₂, GeO₂, CeO₂, TiO₂ und/oder SnO₂.

8. Verwendung nach einem der Ansprüche 1 bis 7, bei der die Glaskeramik 0 bis 7,0, bevorzugt 0 bis 6,5, besonders bevorzugt 1,0 bis 6,5 und ganz besonders bevorzugt 2,0 bis 6,5 Gew.-% P₂O₅ enthält.

9. Verwendung nach einem der Ansprüche 1 bis 8, bei der die Glaskeramik 0 bis 6,0 und bevorzugt 0 bis 5,0 Gew.-% weiteres Oxid fünfwertiger Elemente Me^{V}₂O₅ und/oder 0 bis 6,0 Gew.-% Oxid sechswertiger Elemente Me^{VI}O₃ enthält, wobei Me^{V}₂O₅ insbesondere ausgewählt ist aus V₂O₅, Ta₂O₅ und/oder Nb₂O₅ und Me^{VI}O₃ insbesondere ausgewählt ist aus WO₃ und/oder MoO₃.

10. Verwendung nach einem der Ansprüche 1 bis 9, bei der die Glaskeramik mindestens eine und bevorzugt alle der folgenden Komponenten in den angegebenen Mengen enthält:
| Komponente | Gew.-% |
|---|---|
| SiO₂ | 58,0 bis 92,0 |
| Li₂O | 2,0 bis 10,0 |
| Me^{I}₂O | 0 bis 13,0 |
| Me^{II}O | 0 bis 11,0 |
| Me^{III}₂O₃ | 0 bis 10,0 |
| Me^{IV}O₂ | 0 bis 21,0 |
| P₂O₅ | 0 bis 7,0 |
| Me^{V}₂O₅ | 0 bis 6,0 |
| Me^{VI}O₃ | 0 bis 6,0 |
| Fluor | 0 bis 5,0. |

11. Verwendung nach einem der Ansprüche 1 bis 10, bei der die Glaskeramik 5,0 bis 50,0 und bevorzugt 10,0 bis 30,0 Gew.-% Tiefquarz als Kristallphase enthält.

12. Verwendung nach einem der Ansprüche 1 bis 11, bei der die Glaskeramik Lithiumphosphat und/oder Lithiumsilikat als weitere Kristallphase enthält und insbesondere 5,0 bis 30,0, bevorzugt 10,0 bis 25,0 Gew.-% Lithiumdisilikat enthält.

13. Verwendung von Ausgangsglas, das die Komponenten der Glaskeramik wie in einem der Ansprüche 1 bis 10 definiert enthält und insbesondere Keime für die Kristallisation von Tiefquarz enthält, als Dentalmaterial.

14. Verwendung nach einem der Ansprüche 1 bis 13, wobei die Glaskeramik und das Ausgangsglas in Form von einem Pulver, einer Fritte, einem Rohling oder einer dentalen Restauration vorliegen.

15. Verwendung nach einem der Ansprüche 1 bis 13 zur Herstellung dentaler Restaurationen.

16. Verwendung nach Anspruch 15, wobei der Glaskeramik oder dem Ausgangsglas durch Verpressen, Sintern oder maschinelle Bearbeitung, insbesondere im Rahmen eines CAD/CAM-Verfahrens, die Form der gewünschten dentalen Restauration, insbesondere Brücke, Inlay, Onlay, Veneer, Abutment, Teilkrone, Krone oder Schale, gegeben wird.

17. Verfahren zur Herstellung einer dentalen Restauration, insbesondere Brücke, Inlay, Onlay, Veneer, Abutment, Teilkrone, Krone oder Schale, bei dem der Glaskeramik wie in einem der Ansprüche 1 bis 12 definiert oder dem Ausgangsglas wie in Anspruch 13 definiert durch Verpressen, Sintern oder maschinelle Bearbeitung, insbesondere im Rahmen eines CAD/CAM-Verfahrens, die Form der gewünschten dentalen Restauration gegeben wird.

18. Verfahren nach Anspruch 17, bei dem das Ausgangsglas mindestens einer Wärmebehandlung bei einer Temperatur von 700 bis 950°C für eine Dauer von insbesondere 5 bis 40 min, vorzugsweise 10 bis 30 min, unterworfen wird und der so hergestellten Glaskeramik durch Verpressen, Sintern oder maschinelle Bearbeitung die Form der gewünschten dentalen Restauration gegeben wird.

19. Verfahren nach Anspruch 18, bei dem
(a) Pulver des Ausgangsglases, gegebenenfalls nach Zugabe weiterer Komponenten, zu einem Pulverpressling gepresst wird und
(b) der Pulverpressling einer Wärmebehandlung bei einer Temperatur von 700 bis 950°C für eine Dauer von insbesondere 5 bis 40 min unterworfen wird oder
(a') Schmelze des Ausgangsglases zu einem Glasrohling geformt wird, insbesondere durch Giessen in eine Form, und
(b')der Glasrohling einer Wärmebehandlung bei einer Temperatur von 700 bis 900°C für eine Dauer von insbesondere 5 bis 40 min unterworfen wird.

## Claims

1. Use of glass ceramic, which comprises the following components
| Component | wt.-% |
|---|---|
| SiO₂ | 58.0 to 92.0 |
| Li₂O | 2.0 to 10.0 |
and comprises low quartz as main crystal phase,
as dental material.

2. Use according to claim 1, wherein the glass ceramic comprises 60.0 to 90.0, preferably 70.0 to 83.0 and particularly preferably 73.0 to 81.0 wt.-% SiO₂.

3. Use according to claim 1 or 2, wherein the glass ceramic comprises 2.8 to 9.0, preferably 5.0 to 9.0 and particularly preferably 5.0 to 7.8 wt.-% Li₂O.

4. Use according to any one of claims 1 to 3, wherein the glass ceramic comprises 0 to 13.0, preferably 1.0 to 13.0 and particularly preferably 2.0 to 13.0 wt.-% further alkali metal oxide Me^{I}₂O, wherein Me^{I}₂O is selected in particular from Na₂O, K₂O, Rb₂O and/or Cs₂O.

5. Use according to any one of claims 1 to 4, wherein the glass ceramic comprises 0 to 11.0 and preferably 1.0 to 7.0 wt.-% oxide of divalent elements Me^{II}O, wherein Me^{II}O is selected in particular from MgO, CaO, SrO and/or ZnO.

6. Use according to any one of claims 1 to 5, wherein the glass ceramic comprises 0 to 10.0 and preferably 2.0 to 9.0 wt.-% oxide of trivalent elements Me^{III}₂O₃, wherein Me^{III}₂O₃ is selected in particular from Al₂O₃, B₂O₃, Y₂O₃, La₂O₃, Ga₂O₃ and/or In₂O₃.

7. Use according to any one of claims 1 to 6, wherein the glass ceramic comprises 0 to 21.0 wt.-% further oxide of tetravalent elements Me^{III}O₂, wherein Me^{IV}O₂ is selected in particular from ZrO₂, GeO₂, CeO₂, TiO₂ and/or SnO₂.

8. Use according to any one of claims 1 to 7, wherein the glass ceramic comprises 0 to 7.0, preferably 0 to 6.5, particularly preferably 1.0 to 6.5 and quite particularly preferably 2.0 to 6.5 wt.-% P₂O₅.

9. Use according to any one of claims 1 to 8, wherein the glass ceramic comprises 0 to 6.0 and preferably 0 to 5.0 wt.-% further oxide of pentavalent elements Me^{V}₂O₅ and/or 0 to 6.0 wt.-% oxide of hexavalent elements Me^{VI}O₃, wherein Me^{V}₂O₅ is selected in particular from V₂O₅, Ta₂O₅ and/or Nb₂O₅ and Me^{VI}O₃ is selected in particular from WO₃ and/or MoO₃.

10. Use according to any one of claims 1 to 9, wherein the glass ceramic comprises at least one and preferably all of the following components in the specified amounts:
| Component | wt.-% |
|---|---|
| SiO₂ | 58.0 to 92.0 |
| Li₂O | 2.0 to 10.0 |
| Me^{I}₂O | 0 to 13.0 |
| Me^{II}O | 0 to 11.0 |
| Me^{III}₂O₃ | 0 to 10.0 |
| Me^{IV}O₂ | 0 to 21.0 |
| P₂O₅ | 0 to 7.0 |
| Me^{V}₂O₅ | 0 to 6.0 |
| Me^{VI}O₃ | 0 to 6.0 |
| fluorine | 0 to 5.0. |

11. Use according to any one of claims 1 to 10, wherein the glass ceramic comprises 5.0 to 50.0 and preferably 10.0 to 30.0 wt.-% low quartz as crystal phase.

12. Use according to any one of claims 1 to 11, wherein the glass ceramic comprises lithium phosphate and/or lithium silicate as further crystal phase and in particular comprises 5.0 to 30.0, preferably 10.0 to 25.0 wt.-% lithium disilicate.

13. Use of starting glass, which comprises the components of the glass ceramic as defined in any one of claims 1 to 10 and in particular comprises nuclei for the crystallization of low quartz, as dental material.

14. Use according to any one of claims 1 to 13, wherein the glass ceramic and the starting glass are present in the form of a powder, a frit, a blank or a dental restoration.

15. Use according to any one of claims 1 to 13 for the preparation of dental restorations.

16. Use according to claim 15, wherein the glass ceramic or the starting glass is given the shape of the desired dental restoration, in particular bridge, inlay, onlay, veneer, abutment, partial crown, crown or facet, by pressing, sintering or machining, in particular in a CAD/CAM method.

17. Process for the preparation of a dental restoration, in particular bridge, inlay, onlay, veneer, abutment, partial crown, crown or facet, in which the glass ceramic as defined in any one of claims 1 to 12 or the starting glass as defined in claim 13 is given the shape of the desired dental restoration by pressing, sintering or machining, in particular in a CAD/CAM method.

18. Process according to claim 17, in which the starting glass is subjected to at least one heat treatment at a temperature of 700 to 950°C for a period of in particular from 5 to 40 min, preferably 10 to 30 min, and the so obtained glass ceramic is given the shape of the desired dental restoration by pressing, sintering or machining.

19. Process according to claim 18, in which
(a) powder of the starting glass, optionally after the addition of further components, is pressed to form a powder compact, and
(b) the powder compact is subjected to a heat treatment at a temperature of 700 to 950°C for a period of in particular from 5 to 40 min, or
(a')melt of the starting glass is shaped to form a glass blank, in particular by pouring it into a mould, and
(b') the glass blank is subjected to a heat treatment at a temperature of 700 to 900°C for a period of in particular 5 to 40 min.

## Revendications

1. Utilisation d'une vitrocéramique qui contient les composants suivants :
| Composant | % en poids |
|---|---|
| SiO₂ | de 58,0 à 92,0 |
| Li₂O | de 2,0 à 10,0 |
et qui contient du quartz alpha en tant que phase cristalline principale, en tant que matériau dentaire.

2. Utilisation conforme à la revendication 1, dans laquelle la vitrocéramique contient de 60,0 à 90,0 %, de préférence de 70,0 à 83,0 %, et mieux encore de 73,0 à 81,0 % en poids de SiO₂.

3. Utilisation conforme à la revendication 1 ou 2, dans laquelle la vitrocéramique contient de 2,8 à 9,0 %, de préférence de 5,0 à 9,0 %, et mieux encore de 5,0 à 7,8 % en poids de Li₂O.

4. Utilisation conforme à l'une des revendications 1 à 3, dans laquelle la vitrocéramique contient de 0 à 13,0 %, de préférence de 1,0 à 13,0 %, et mieux encore de 2,0 à 13,0 % en poids d'un autre oxyde de métal alcalin Me^{I}₂O, cet oxyde Me^{I}₂O étant choisi, en particulier, parmi Na₂O, K₂O, Rb₂O et/ou Cs₂O.

5. Utilisation conforme à l'une des revendications 1 à 4, dans laquelle la vitrocéramique contient de 0 à 11,0 %, et de préférence de 1,0 à 7,0 % en poids d'un oxyde d'élément divalent Me^{II}O, cet oxyde Me^{II}O étant choisi, en particulier, parmi MgO, CaO, SrO et/ou ZnO.

6. Utilisation conforme à l'une des revendications 1 à 5, dans laquelle la vitrocéramique contient de 0 à 10,0 %, et de préférence de 2,0 à 9,0 % en poids d'un oxyde d'élément trivalent Me^{III}₂O₃, cet oxyde Me^{III}₂O₃ étant choisi, en particulier, parmi Al₂O₃, B₂O₃, Y₂O₃, La₂O₃, Ga₂O₃ et/ou In₂O₃.

7. Utilisation conforme à l'une des revendications 1 à 6, dans laquelle la vitrocéramique contient de 0 à 21,0 % en poids d'un autre oxyde d'élément tétravalent Me^{IV}O₂, cet oxyde Me^{IV}O₂ étant choisi, en particulier, parmi ZrO₂, GeO₂, CeO₂, TiO₂ et/ou SnO₂.

8. Utilisation conforme à l'une des revendications 1 à 7, dans laquelle la vitrocéramique contient de 0 à 7,0 %, de préférence de 0 à 6 ,5 %, mieux encore de 1,0 à 6,5 % et surtout de 2,0 à 6,5 % en poids de P₂O₅.

9. Utilisation conforme à l'une des revendications 1 à 8, dans laquelle la vitrocéramique contient de 0 à 6,0 % et de préférence de 0 à 5,0 % en poids d'un autre oxyde d'élément pentavalent Me^{V}₂O₅ et/ou de 0 à 6,0 % en poids d'un oxyde d'élément hexavalent Me^{VI}O₃, cet oxyde Me^{V}₂O₅ étant choisi, en particulier, parmi V₂O₅, Ta₂O₅ et/ou Nb₂O₅, et cet oxyde Me^{VI}O₃ étant choisi, en particulier, parmi WO₃ et/ou MoO₃.

10. Utilisation conforme à l'une des revendications 1 à 9, dans laquelle la vitrocéramique contient au moins l'un des composants suivants, et de préférence tous ces composants, en les proportions indiquées :
| Composant | % en poids |
|---|---|
| SiO₂ | de 58,0 à 92,0 |
| Li₂O | de 2,0 à 10,0 |
| Me^{I}₂O | de 0 à 13,0 |
| Me^{II}O | de 0 à 11,0 |
| Me^{III}₂O₃ | de 0 à 10,0 |
| Me^{IV}O₂ | de 0 à 21,0 |
| P₂O₅ | de 0 à 7,0 |
| Me^{V}₂O₅ | de 0 à 6,0 |
| Me^{VI}O₃ | de 0 à 6,0 |
| Fluor | de 0 à 5,0. |

11. Utilisation conforme à l'une des revendications 1 à 10, dans laquelle la vitrocéramique contient de 5,0 à 50,0 % et de préférence de 10,0 à 30,0 % en poids de quartz alpha en tant que phase cristalline.

12. Utilisation conforme à l'une des revendications 1 à 11, dans laquelle la vitrocéramique contient du phosphate de lithium et/ou du silicate de lithium en tant qu'autre phase cristalline, et en particulier, contient de 5,0 à 30,0 % et de préférence de 10,0 à 25,0 % en poids de disilicate de lithium.

13. Utilisation d'un verre de départ, qui contient les composants d'une vitrocéramique telle que définie dans l'une des revendications 1 à 10 et qui contient, en particulier, des germes de cristallisation du quartz alpha, en tant que matériau dentaire.

14. Utilisation conforme à l'une des revendications 1 à 13, dans laquelle la vitrocéramique ou le verre de départ se présente sous forme d'une poudre, d'une fritte, d'une ébauche ou d'une restauration dentaire.

15. Utilisation conforme à l'une des revendications 1 à 13 pour la fabrication de restaurations dentaires.

16. Utilisation conforme à la revendication 15, dans laquelle on donne à la vitrocéramique ou au verre de départ, par compression, frittage ou travail à la machine, en particulier dans le cadre d'un procédé CAO/FAO, la forme de la restauration dentaire voulue, en particulier celle d'un bridge, d'un insert in-lay ou onlay, d'une facette, d'un pilier, d'une couronne partielle, d'une couronne ou d'une coquille.

17. Procédé de fabrication d'une restauration dentaire, en particulier d'un bridge, d'un insert in-lay ou onlay, d'une facette, d'un pilier, d'une couronne partielle, d'une couronne ou d'une coquille, dans lequel on donne à une vitrocéramique telle que définie dans l'une des revendications 1 à 12 ou à un verre de départ tel que défini dans la revendication 13, par compression, frittage ou travail à la machine, en particulier dans le cadre d'un procédé CAO/FAO, la forme de la restauration dentaire voulue.

18. Procédé conforme à la revendication 17, dans lequel on fait subir au verre de départ au moins un traitement thermique, à une température de 700 à 950 °C et durant en particulier de 5 à 40 minutes et de préférence de 10 à 30 minutes, et l'on donne à la vitrocéramique ainsi fabriquée, par compression, frittage ou travail à la machine, la forme de la restauration dentaire voulue.

19. Procédé conforme à la revendication 18, dans lequel
a) on comprime une poudre de verre de départ, après addition, le cas échéant, d'autres composants, pour en faire un comprimé de poudre,
b) et l'on fait subir à ce comprimé de poudre un traitement thermique, à une température de 700 à 950 °C et durant en particulier de 5 à 40 minutes,
ou bien
a') on façonne, en particulier par coulée dans un moule, une masse fondue de verre de départ pour en faire une ébauche en verre,
b') et l'on fait subir à cette ébauche en verre un traitement thermique, à une température de 700 à 900 °C et durant en particulier de 5 à 40 minutes.
